(19) Europäisches Patentamt
European Patent Office
Office européen des brevets

(11) EP 4 548 962 A1

(12) EUROPEAN PATENT APPLICATION

(43) Date of publication:
07.05.2025 Bulletin 2025/19

(51) International Patent Classification (IPC):
A61N 1/36 (2006.01)

(21) Application number: 23207588.7

(22) Date of filing: 03.11.2023

(52) Cooperative Patent Classification (CPC):
A61N 1/3603; A61N 1/36142; A61N 1/3614

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Stichting IMEC Nederland
5656 AE Eindhoven (NL)

(72) Inventors:
• XIN, Haoming
  5655 BN Eindhoven (NL)
• VAN WEGBERG, Roland
  5345 BZ Oss (NL)
• ZHOU, Meiyi
  5611 BM Eindhoven (NL)

(74) Representative: AWA Sweden AB
Box 5117
200 71 Malmö (SE)

(54) **A STIMULATOR DEVICE FOR PROVIDING A STIMULATION SIGNAL, A SYSTEM FOR PROVIDING TEMPORAL INTERFERENCE STIMULATION, AND A METHOD FOR MONITORING A STIMULATION SIGNAL**

(57) A stimulator device (102a; 102b; 102c; 102d) for providing a stimulation signal comprises: a stimulation signal generating unit (110) configured to generate a stimulation current signal and provide the stimulation current signal to an output (130) of the stimulation signal generating unit (110), wherein the stimulation current signal comprises a sinusoidal-like waveform; and an impedance monitoring unit (160) connected to the output (130) of the stimulation signal generating unit (110), wherein the impedance monitoring unit (160) is configured to, while the stimulation current signal is provided on the output (130) of the stimulation signal generating unit (110), determine an extreme voltage at an extreme point of a voltage signal at the output (130) and a phase delay between the voltage signal and the stimulation current signal for determining information of an impedance relating to an interface between an electrode (10a; 10b; 10c; 10d), connected to the output (130), and tissue.

Fig. 1

**Description**

Technical field

The present description relates to a stimulator device and, in particular, a stimulator device which may be part of a system for providing temporal interference stimulation of a brain and/or a nerve. The present description also relates to a method for monitoring a stimulation signal.

Background

Electrical stimulation is widely used in clinical practice. Stimulation may typically be delivered using biphasic charge balanced pulses.

When an accurate spatial selectivity of stimulation is desired, interferential stimulation based on two or more stimulation signals may be used. Interference of the two or more stimulation signals may thus generate an interferential stimulation signal within tissue, which allows selectively stimulating a location within the tissue. The stimulation signals for forming the interferential stimulation may be continuously provided for a long period of time.

An interface between an electrode and tissue, for providing a stimulation signal into the tissue, may change over time. This may affect the electrical stimulation and/or relate to safety issues for providing the electrical stimulation.

Thus, there is a need to acquire information of the electrode-tissue interface and, in particular, to acquire information during output of a stimulation signal since the stimulation signal may be continuously provided for a long period of time.

Summary

An objective of the present description is to enable acquiring of information of an electrode-tissue interface used for providing a stimulation signal into tissue. A particular objective of the present description is to enable acquiring of the information during output of the stimulation signal.

These and other objectives are at least partly met by the invention as defined in the independent claims. Preferred embodiments are set out in the dependent claims.

According to a first aspect, there is provided a stimulator device for providing a stimulation signal, said stimulator device comprising: a stimulation signal generating unit configured to generate a stimulation current signal and provide the stimulation current signal to an output of the stimulation signal generating unit, wherein the stimulation current signal comprises a sinusoidal-like waveform; and an impedance monitoring unit connected to the output of the stimulation signal generating unit, wherein the impedance monitoring unit is configured to, while the stimulation current signal is provided on the output of the stimulation signal generating unit, determine an extreme voltage at an extreme point of a voltage signal at the output and a phase delay between the voltage signal and the stimulation current signal for determining information of an impedance relating to an interface between an electrode, connected to the output, and tissue.

Thanks to the stimulator device of the first aspect, impedance of the electrode-tissue interface can be determined. The information of the impedance is determined based on the impedance monitoring unit being connected to the output of the stimulation signal generating unit. Thus, there is no need for additional electrodes for acquiring signals that are to be used for determining the impedance. Rather, the impedance may be directly determined at the output of the stimulation signal generating unit.

In addition, the stimulation signal generating unit is configured to generate a current signal, whereas the impedance monitoring unit is configured to determine a voltage. Thus, the impedance monitoring unit may determine the voltage while the stimulation current signal is provided on the output of the stimulation generating unit. In particular, the stimulator device is configured to utilize the stimulation current signal for determining information of impedance, such that a dedicated signal for determining impedance need not be used but rather the stimulation current signal used for providing a stimulation may also be used for determining information of impedance. Thus, information of impedance may be determined in an energy efficient manner.

The extreme voltage at the extreme point of the voltage signal and the phase delay between the voltage signal and the stimulation current signal may be used for determining a complex impedance (including resistive and capacitive values) of the interface between the electrode and tissue. The determining of the complex impedance may further use known information of characteristics of the stimulation current signal, such as using information of amplitude and carrier frequency of the stimulation current signal.

The information of the impedance of the interface between the electrode and tissue may be used in numerous ways. The impedance may provide clinical information to a doctor. For instance, for an implanted device, tissue may grow around an electrode that is implanted in a body. The impedance of the interface between the electrode and tissue may thus provide a measure relating to tissue growth around the electrode.

# EP 4 548 962 A1

The impedance between the electrode and tissue will affect a supply voltage needed by the stimulation signal generating unit for providing a desired amplitude of the stimulation current signal. The information of the impedance may thus be used for controlling a supply voltage so as to allow saving power in generating of the stimulation current signal.

The information of the impedance of the interface between the electrode and tissue may be used for determining whether the electrode is properly arranged in contact with tissue. Thus, information of impedance may be used for monitoring that the electrode is properly arranged and may be used for identifying if the electrode is affected so as to lose proper contact with tissue.

In addition, impedance between an electrode and a nerve may be slightly affected when an action potential is triggered inside the nerve. Thus, by monitoring information of the impedance of the interface between the electrode and tissue, the stimulator device may be able to determine if the stimulation current signal is successful in triggering an action potential is the nerve. This may further be used for adjusting control of a stimulation current signal to be output by the stimulation signal generating unit.

The stimulator device may be particularly useful for generating stimulation signals that are to be used for temporal interference stimulation, wherein multiple stimulation signals interfere for causing an interferential signal within a brain and/or a nerve to be stimulated, such that the interferential signal may provide a desired stimulation. Thus, a plurality of stimulator devices may be used, each dedicated to generating one stimulation signal that is to be used for forming the temporal interference stimulation. The temporal interference stimulation allows spatially selecting a location to be stimulated, such as a location in a brain and/or a nerve of a living being, such as a human being or an animal.

The stimulation current signal having a sinusoidal-like waveform implies that the stimulation current signal may be sinusoidal but does not necessarily need to be a pure sinusoidal waveform. For instance, the stimulation current signal may be a pseudo-sinusoidal waveform, a modified sinusoidal waveform, or a multi-level waveform approximating a sinusoidal waveform. The stimulation current signal may further be an alternating current (AC) signal.

The stimulation current signal may be a continuous signal, which is provided over a relatively long period of time. However, the stimulation current signal may be a sequence of temporally separated bursts, wherein each burst comprises the sinusoidal-like waveform. Using bursts, power consumption may be limited since the stimulation current signal need not be constantly output, while a corresponding effect on tissue, such as a brain and/or a nerve, of the stimulation signal compared to a continuous stimulation signal may still be achieved.

The stimulation current signal may be used for stimulating a brain and/or a nerve of a living being. For instance, the stimulation current signal may be used for triggering a neural signal to be generated in the brain and/or the nerve or for blocking a neural signal that is propagating in the brain and/or the nerve. Temporal interferential stimulation may be used for accurately controlling a spatial location in the brain and/or the nerve in which the stimulation is provided.

The stimulation signal generating unit may output a current signal. The current signal may be converted to a voltage signal by being output to an electrode connected to tissue. Thus, the impedance monitoring unit may be configured to monitor a voltage signal based on a stimulation signal which is a current signal.

The voltage signal varies with time and comprises extreme points in a time-dependent signal. An extreme point is a point of the voltage signal at a particular time instance when the voltage signal has a local maximum value or local minimum value, and a time derivative of the voltage signal is zero. The extreme voltage is thus a voltage value at the extreme point.

The extreme voltage may be referred to herein as a peak voltage, which corresponds to a local maximum value of the voltage signal. However, it should be realized that, when discussion of peak voltage is made herein, a local minimum value of the voltage signal may alternatively be used.

According to an embodiment, the stimulator device further comprises an electrode connected to the output of the signal generating unit for receiving the stimulation current signal, said electrode being configured for connection to tissue for providing the stimulation current signal into a body part.

The electrode may be configured to be implanted, such that the electrode may be connected to tissue within a living being, such as connected to a wall of a nerve within the living being. However, the electrode may alternatively be configured to be arranged in contact with the living being, externally to the living being, such as arranged in contact with skin. It should be further realized that the electrode may not necessarily be in direct contact with the tissue. For instance, a gel may be provided between the electrode and the tissue in order to improve electrical contact between the electrode and the tissue.

The body part may for instance be a brain and/or a nerve of a living being, such that the stimulator device is configured to provide the stimulation signal for stimulating the brain and/or the nerve.

It should be realized that the stimulation signal generating unit and the impedance monitoring unit may be packaged together for providing generation of a stimulation current signal and impedance monitoring within a compact device. The stimulator device may further comprise the electrode, such that the stimulation signal generating unit and the impedance monitoring unit may be delivered together with the electrode. However, it should be realized that the electrode may alternatively be separately delivered and that the stimulation signal generating unit and the impedance monitoring unit may be connected to the electrode when the stimulator device is to be used.

3

According to an embodiment, the stimulator device further comprises a processor configured to receive the extreme voltage and the phase delay and configured to determine the impedance based on the received extreme voltage and phase delay and knowledge of an amplitude and frequency of the stimulation current signal.

This implies that the stimulator device is configured to determine the impedance within a processor that is part of the stimulator device. For instance, the processor may be arranged in a common housing with the stimulation signal generating unit and the impedance monitoring unit. This may ensure that the impedance may be determined within a compact device and that impedance may be determined in a processor that is physically close to the stimulation signal generating unit. Hence, use of the determined impedance for real-time control of the stimulator device is facilitated.

The processor may have knowledge of the amplitude and frequency of the stimulation signal. Information of the amplitude and frequency of the stimulation signal may be stored such that the processor has access to the information.

The processor may be configured to provide a control signal to the stimulation signal generating unit. This implies that the processor has knowledge of the amplitude and frequency of the stimulation signal based on the processor being configured to control these characteristics of the stimulation signal being generated by the stimulation signal generating unit.

The processor and the stimulation signal generating unit may be configured to communicate with each other. Thus, the stimulation signal generating unit may communicate information of the amplitude and frequency of the stimulation signal to the processor.

The stimulation signal generating unit, the impedance monitoring unit, the electrode, and the processor may be arranged in a compact device. The compact device may thus be able to generate the stimulation signal, provide an interface for output of the stimulation signal to tissue, and determine the impedance of the interface.

However, according to an alternative, the stimulation signal generating unit and the impedance monitoring unit, the electrode, and the processor may form parts of a stimulator system, wherein components are arranged in separate physical units.

According to an embodiment, the stimulator device further comprises a compensation unit configured to generate a compensation current signal for charge balancing and to provide the compensation current signal to the output of the stimulation signal generating unit for compensating an unbalanced charge of the stimulation current signal and forming a compensated stimulation signal at the output of the stimulation signal generating unit.

A mismatch between positive and negative parts of a waveform may cause an unbalanced charge to electrodes connected to the stimulator circuit. The unbalanced charge may introduce an offset voltage build-up onto the electrodes over time, in particular as the interface between the electrode and tissue is often very capacitive. If the stimulator device is used to continuously output a stimulation signal, such as a burst of the stimulation signal, for a long period of time, the unbalanced charge can cause safety issues to the electrode and/or the tissue. For instance, the offset voltage build-up may form such a large electrical field that electrical breakdown of water occurs. The offset voltage build-up may also cause the electrode to break such that a subject to the stimulation may be exposed to toxic substances.

The compensation unit may be configured to provide the compensation current signal during output of the stimulation current signal. Thanks to the compensation current signal provided by the compensation unit, any unbalanced charge may thus be compensated during output of the stimulation signal, such as during a burst of the stimulation signal. Thus, the stimulator device may allow the burst to be output continuously for a long period of time without any need to interrupt stimulation in order for charge balance compensation to be performed.

By using the compensation unit, the stimulator device may ensure that charge balancing is provided so as to compensate for any mismatch between positive and negative parts of the waveform of the stimulation signal. This implies that the stimulator device may provide compensation instead of (or in combination with) control of functionality of components of the stimulator device.

The stimulation signal generating unit may comprise components having nonideal function which may cause an unbalance in the waveform. If the stimulation signal generating unit is to be provided in an integrated circuit, compensation of nonideal function of components may be difficult or impossible to achieve in the integrated circuit. Thanks to providing the compensation unit, the stimulator device may still ensure that an unbalanced charge is compensated to improve safety of the stimulator circuit. Hence, the stimulation signal generating unit may be provided in an integrated circuit providing a very small form factor of the stimulation signal generating unit.

The stimulator device may be particularly useful for generating stimulation signals that are to be used for temporal interference stimulation, wherein multiple stimulation signals interfere for causing an interferential signal within a brain and/or a nerve to be stimulated, such that the interferential signal may provide a desired stimulation.

In temporal interference stimulation, stimulation may be provided continuously over relatively long time and/or with a large number of bursts of individual stimulation signals. In order to avoid crosstalk between different stimulation signals, independent current source and current sink may be used for each stimulator device. This implies that there is a high risk of mismatch between the current source and the current sink such that build-up of an offset voltage onto electrodes may be likely. Thus, use of the compensation unit may ensure that offset voltage build-up in temporal interference stimulation is avoided.

The stimulation signal is provided at the output of the stimulation signal generating unit. Based on the stimulation signal, the compensation current signal is generated and also provided at the output of the stimulation signal generating unit. Thus, the compensation current signal is combined with the stimulation signal at the output to define the compensated stimulation signal. Hence, forming of the compensated stimulation signal may involve combining the stimulation signal at the output of the stimulation signal generating unit with the compensation current signal.

According to an embodiment, the compensation unit comprises an extreme voltage detector for detecting the extreme voltage of the voltage signal, wherein the impedance monitoring unit is configured to receive the detected extreme voltage from the compensation unit for determining the extreme voltage.

The compensation unit may be configured to determine the compensation current signal based on a common mode voltage of the stimulation signal. Thus, the compensation unit may be configured to detect the common mode voltage in order to detect presence of an unbalanced charge, which may need to be compensated for by the compensation unit.

Thus, the compensation unit may be configured to perform extreme point detection of the stimulation signal for determining an unbalance of the common mode voltage based on the stimulation signal. This implies that the compensation unit may be configured to detect the extreme voltage, which is also needed by the impedance monitoring unit. Hence, the extreme voltage can be used both by the compensation unit and the impedance monitoring unit and needs only be detected by one of the units.

It should be realized that if the stimulation current signal generated by the stimulation signal generating unit has a relatively short duration, no compensation unit may be needed.

According to an embodiment, the impedance monitoring unit comprises a counter for determining a time delay representative of the phase delay.

The counter may be configured to determine a time delay between corresponding points on the stimulation current signal and the voltage signal at the output. The counter may thus determine the time delay between corresponding points on waveforms. Using knowledge of frequency of the stimulation current signal, the time delay may be converted to a phase. This conversion need not necessarily be performed by the impedance monitoring unit. Rather, the impedance monitoring unit may be configured to output the time delay as a representation of the phase delay.

The counter may be configured to receive a trigger signal from the stimulation signal generating unit. For instance, the stimulation signal generating unit may be configured to generate the stimulation current signal in a controlled manner, such that a trigger may be provided to the counter corresponding to an extreme point, such as a peak, of the stimulation current signal. The counter may thus start counting based on receipt of the trigger.

The counter may further receive a trigger to stop counting based on detection of the extreme voltage of the voltage signal. Thus, the counter may be configured to determine the time delay based on determining a time period between the extreme point of the stimulation current signal and the following extreme point of the voltage signal.

According to an embodiment, the impedance monitoring unit being configured to determine the extreme voltage comprises the impedance monitoring unit being configured to determine both an upper extreme voltage at an upper extreme point of the voltage signal and a lower extreme voltage at a lower extreme point of the voltage signal.

This may be used for improving resolution of determining the impedance based on the information acquired by the impedance monitoring unit.

The upper extreme voltage and the lower extreme voltage may be used together with a phase delay associated with each of the upper extreme voltage and the lower extreme voltage for determining the impedance of the interface between the electrode and tissue.

The upper extreme point corresponds to a local maximum of the voltage signal. The upper extreme voltage is a value of the voltage signal at the upper extreme point. The lower extreme point corresponds to a local minimum of the voltage signal. The lower extreme voltage is a value of the voltage signal at the lower extreme point.

The processor may be configured to receive the upper extreme voltage and the lower extreme voltage from the impedance monitoring unit. The processor may further be configured to receive phase delays associated with each of the upper extreme voltage and the lower extreme voltage. The processor may be configured to use this information for determining the impedance with a high resolution.

According to an embodiment, the stimulation current signal comprises a sequence of temporally separated bursts, wherein each burst comprises an alternating current (AC) signal having the sinusoidal-like waveform.

The stimulation current signal comprising bursts may be suitable for use in temporal interference stimulation, wherein multiple bursts in a sequence may provide a desired stimulating effect in tissue, such as in a brain and/or a nerve. The use of bursts imply that power consumption may be limited since the stimulation current signal need not be constantly output over a long period of time. In addition, the use of bursts also allows compensation for unbalanced charges that may build up over a continuous output of a stimulation current signal, such that safety issues related to build-up of charges at the interface between the electrode and tissue may be avoided.

The impedance monitoring unit is configured to determine the extreme voltage of the voltage signal and the phase delay, while a burst of the stimulation current signal is provided on the output.

According to an embodiment, the stimulator device is configured to, between bursts of the stimulation current signal, reset a direct current (DC) voltage at the output of the stimulation signal generating unit to a reference voltage.

After a burst of the stimulation current signal, a remaining offset voltage may be present at the output of the stimulation signal generating unit. The remaining offset voltage may be present even if a compensation unit is used for compensating unbalanced charges during output of a burst.

Thanks to the stimulator device being configured to reset the DC voltage at the output, safety issues related to build-up of charges at the interface between the electrode and tissue may be avoided.

According to an alternative, the remaining offset voltage may be compensated for by controlling a reference voltage being used by the compensation unit for determining the compensation current signal. The reference voltage may be adjusted based on the remaining offset voltage after a burst such that the compensation current signal generated by the compensation unit during a next burst will compensate for the remaining offset voltage.

According to an embodiment, the stimulator device further comprises a power management unit configured to control a supply voltage controlling generation of the stimulation current signal by the stimulation signal generating unit based on the determined extreme voltage.

The supply voltage may define a maximum available voltage level of the voltage signal formed at the output of the stimulation signal generating unit. However, the voltage level of the voltage signal may depend on an amplitude to be used by the stimulation signal and on impedance of the interface between the electrode and tissue. Thus, if no power management unit is used, the supply voltage should be sufficient to support a worst-case scenario of largest amplitude of the stimulation current signal and largest impedance.

The power management unit may be configured to set a supply voltage that supports the determined extreme voltage, possibly with a small margin. Thus, the supply voltage may be controlled such that power consumption is saved by avoiding use of an unnecessarily high supply voltage.

The extreme voltage may be determined for a burst in the stimulation signal. The power management unit may then control the supply voltage that is used for the next burst.

The power management unit may also be configured to control a reference voltage. The reference voltage may provide a DC voltage level, such that the stimulation current signal may apply the sinusoidal-like waveform superposed on the DC voltage level. The reference voltage may thus be used for resetting a voltage at the output of the stimulation signal generating unit to the reference voltage.

According to a second aspect, there is provided a system for providing temporal interference stimulation of a brain and/or a nerve of a living being, said system comprising: a first pair of electrodes configured to be arranged in a first relation to the brain and/or the nerve; a second pair of electrodes configured to be arranged in a second relation to the brain and/or the nerve; wherein each electrode of the first pair of electrodes and the second pair of electrodes forms part of a respective stimulator device according to the first aspect.

Effects and features of this second aspect are largely analogous to those described above in connection with the first aspect. Embodiments mentioned in relation to the first aspect are largely compatible with the second aspect.

Thus, a plurality of stimulator devices may be part of a system for providing stimulation of a brain and/or a nerve of a living being, such as a human being or an animal. Each stimulator device may be used for providing a stimulation current signal at a respective electrode.

The system is able to monitor impedance of the electrode-tissue interface for each of the electrodes. This may provide clinical information relating to the electrode-tissue interface. In addition, the system may use the impedance information for saving power in generating of the stimulation current signals to be provided to the respective electrodes.

A first and a second pair of stimulator devices may be used for output of stimulation signals to a first and a second pair of electrodes. A first stimulator device may thus be combined with a second stimulator device to form the first pair of stimulator devices and a third stimulator device may be combined with a fourth stimulator device to form the second pair of stimulator devices. The first stimulator device and the second stimulator device may be configured to receive an identical current waveform, which may be copied and optionally amplified by a current source and a current sink. The first stimulator device and the second stimulator device may be controlled such that the first stimulator device may enable the current source when the second stimulator device enables the current sink, and vice versa. Each of the first stimulator device and the second stimulator device may be connected to a respective electrode of the first pair of electrodes which may thus receive stimulation current signals that are in anti-phase for forming a stimulation signal to be transmitted into the brain and/or the nerve.

The third stimulator device and the fourth stimulator device may generate stimulation current signals in the same way as described above for the first stimulator device and the second stimulator device. Each of the third stimulator device and the fourth stimulator device may be connected to a respective electrode of the second pair of electrodes which may thus receive stimulation current signals that are in anti-phase for forming a stimulation signal to be transmitted into the brain and/or the nerve.

It should further be realized that the system may comprise a set of electrodes, such that the electrode to be used for transmitting of the stimulation current signal into the brain and/or nerve may be dynamically selected for each stimulator device when the system is used.

It should be realized that the system may comprise more than two pairs of stimulator devices for forming more than two stimulation signals in the tissue. A number of stimulator devices of the system may be dependent on the stimulation to be provided. For instance, in some embodiments, the system may comprise more than two pairs of stimulator devices for generating more than two stimulation signals for causing an interferential stimulation signal to be formed based on more than two stimulation signals.

According to an embodiment, the system further comprises a reference electrode for defining a reference voltage in relation to each electrode of the first pair of electrodes and the second pair of electrodes.

Thus, a separate reference voltage may be provided to the tissue. The reference electrode provides a bias voltage to tissue.

According to an embodiment, the system comprises a common power management unit for controlling a common supply voltage of the stimulation signal generating units of each of the stimulator devices.

Thus, the stimulation signal generating units may receive a common supply voltage. This implies that a single supply voltage may be controlled for providing power saving in the system.

According to an alternative embodiment, a respective supply voltage controlling generation of the stimulation current signal by the respective stimulation signal generating units is individually controlled.

Thus, each stimulation signal generating unit of each of the stimulator devices in the system may receive an individually controlled supply voltage. Thus, different supply voltages may be provided to different stimulation signal generating units. This implies that power may be saved individually for each of the stimulation signal generating units, such that more power may be saved compared to using a common supply voltage.

The supply voltages for each of the stimulation signal generating units may be determined by a common power management unit. Thus, a single power management unit may be used, which receives input for determining the supply voltages for each of the stimulation signal generating units. The single power management unit may thus provide a plurality of output signals, each output signal being dedicated for a unique stimulation signal generating unit.

The power management unit may also be configured to control a reference voltage. The reference voltage may provide a DC voltage level, such that the stimulation current signal may apply the sinusoidal-like waveform superposed on the DC voltage level. The reference voltage may thus be used for resetting a voltage at the output of the stimulation signal generating unit to the reference voltage.

The reference voltage may also be applied to the reference electrode. Since all of the electrodes of the system may be related to the common reference voltage, the power management unit may control a single reference voltage which may be used by each of the stimulator devices.

According to a third aspect, there is provided a method for monitoring a stimulation signal, said method comprising: generating a stimulation current signal and providing the stimulation current signal to an output of a stimulation signal generating unit, wherein the stimulation current signal comprises a sinusoidal-like waveform; while the stimulation current signal is provided on the output of the stimulation signal generating unit, determining an extreme voltage at an extreme point of a voltage signal at the output and a phase delay between the voltage signal and the stimulation current signal for determining information of an impedance relating to an interface between an electrode, connected to the output, and tissue.

In particular, the method may be performed in the stimulator device of the first aspect or in the system according to the second aspect.

Effects and features of this third aspect are largely analogous to those described above in connection with the first and second aspects. Embodiments mentioned in relation to the first and second aspects are largely compatible with the third aspect.

The method is able to monitor impedance of the electrode-tissue interface for an electrode, while a stimulation current signal is output. This may provide clinical information relating to the electrode-tissue interface. In addition, the impedance information may be used for saving power in generating of the stimulation current signals to be provided at the output.

Brief description of the drawings

The above, as well as additional objects, features, and advantages of the present description, will be better understood through the following illustrative and non-limiting detailed description, with reference to the appended drawings. In the drawings like reference numerals will be used for like elements unless stated otherwise.

Fig. 1 is a schematic view of a system according to a first embodiment.
Fig. 2 is a schematic view of stimulation current signals with power management of the system according to the first embodiment.
Fig. 3 is a schematic view of a system according to a second embodiment.

Fig. 4 is a schematic view of stimulation current signals with power management of the system according to the second embodiment.

Fig. 5 is a flow chart of a method according to an embodiment.

Detailed description

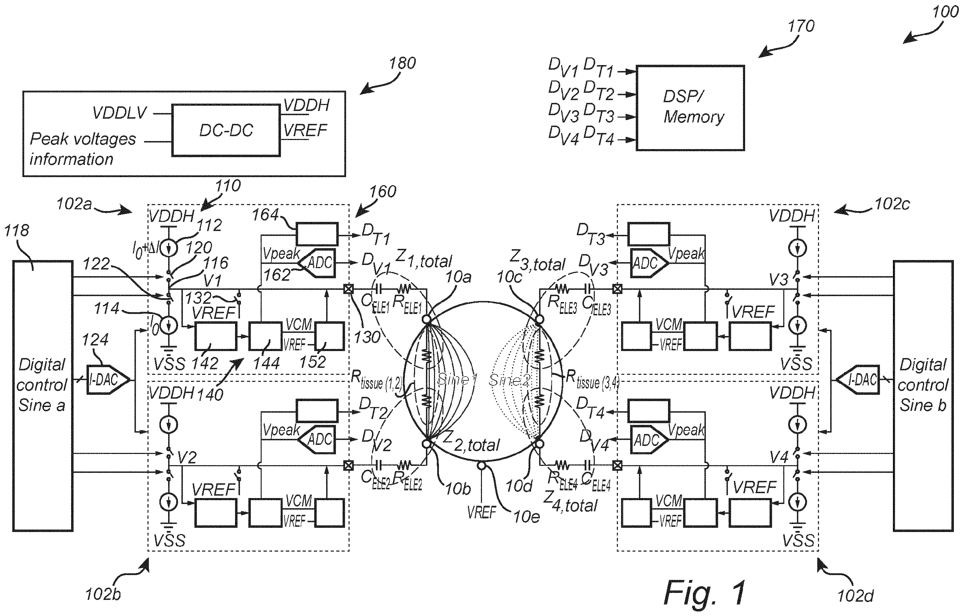

In Fig. 1, a system 100 for providing temporal interference stimulation of a brain and/or a nerve according to a first embodiment is shown. The system 100 comprises a plurality of stimulator devices 102a, 102b, 102c, 102d. Below, mainly a single stimulator device 110a will be described in detail for simplicity and brevity. In addition, it should be understood that the stimulator device 102a may be used in a different system. The stimulator device 102a may be used as a sole stimulator device 102a providing a stimulation signal. It should further be understood that each of the stimulator devices 102a, 102b, 102c, 102d may be implemented in a same manner, such that the description of the stimulator device 102a applies as well to the other stimulator devices 102b, 102c, 102d.

The stimulator device 102a comprises a stimulation generating unit 110 configured to generate a stimulation signal. The stimulation generating unit 110 may comprise a current source 112 and a current sink 114. The current source 112 may be connected between a supply voltage and an output node 116 and the current sink 114 may be connected between ground and the output node 116.

The stimulation generating unit 110 may further comprise a stimulation controller 118 which may provide digital control of a first switch 120 arranged between the current source 112 and the output node 116 and a second switch 122 arranged between the current sink 114 and the output node 116. The stimulation controller 118 may thus control which of the current source 112 and the current sink 114 is connected to the output node 116.

The current source 112 and the current sink 114 may thus form independent components for generating a stimulation current signal at the output node 116. The use of independent components may avoid crosstalk between the stimulator devices 102a, 120b, 102c, 102d, which may be used for example for providing interferential stimulation, during output of a stimulation signal at the output node 116.

The output node 116 may further be connected to an output 130 of the stimulation signal generating unit 110. The output 130 may further be connected, e.g., to an electrode 10 for providing the stimulation signal to a living being. It should be realized that the electrode 10a may not necessarily be part of the stimulator device 102a.

The stimulation signal generating unit 110 may be configured to provide the stimulation signal via the output node 116 to the output 130 of the stimulation signal generating unit 110. The stimulation signal may be a sinusoidal-like waveform, such as a pure sinusoidal waveform, a pseudo-sinusoidal waveform, a modified sinusoidal waveform, or a multi-level waveform approximating a sinusoidal waveform.

For instance, the stimulation signal generating unit 110 may be utilized in temporal interference stimulation. Temporal interference stimulation uses multiple stimulator circuits for providing multiple stimulation signals having sinusoidal-like waveforms, wherein interference between the multiple stimulation signals allows spatially selecting a location for stimulation in a brain and/or a nerve of a living being. In particular, a maximum amplitude of the interferential stimulation signal may be provided inside the brain and/or the nerve such that stimulation may be provided deep within the brain and/or a nerve by signals output by electrodes arranged externally to the brain and/or the nerve.

Temporal interference stimulation may be provided by duty-cycled stimulation signals, such that the stimulation signal may comprise a sequence of temporally separated bursts, wherein each burst comprises a sinusoidal-like waveform. For instance, a stimulation signal having bursts with a duration in a range of 10 $\mu$s - 5 ms may be used.

As shown in Fig. 1, two pairs of stimulator devices 102a, 102b, 102c, 102d may be used for generating two sinusoidal stimulation current signals in the brain and/or the nerve. Thus, a first pair of a first stimulator device 102a and a second stimulator device 102b may generate a first stimulation current between a first electrode 10a and a second electrode 10b. Further, a second pair of a third stimulator device 102c and a fourth stimulator device 102d may generate a second stimulation current between a third electrode 10c and a fourth electrode 10d.

The first, second, third, and fourth electrodes 10a, 10b, 10c, 10d are arranged at different locations in relation to tissue for providing temporal interferential stimulation in a body part. As illustrated in Fig. 1, the electrodes 10a, 10b, 10c, 10d may be arranged at different circumferential locations around a cross-section of a nerve for providing temporal interferential stimulation in the nerve. In addition, a reference electrode 10e may be provided and may be connected . The reference electrode 10e may be arranged at yet another location in relation to tissue. The reference electrode 10e may further be configured to receive a reference voltage for providing a bias voltage in tissue.

The first stimulator device 102a and the second stimulator device 102b may be configured to receive an identical voltage waveform. The voltage waveform may be generated by the stimulation controller 118 and may be converted to a current waveform by a current digital-to-analog converter 124, which may further output the current waveform to the first stimulator device 102a and the second stimulator device 102b. For the first stimulator device 102a, the current waveform may be copied and optionally amplified by the current source 112 and the current sink 114. The stimulation controller 118 further controls the first stimulator device 102a for enabling the first switch 120 or the second switch 122 for enabling the current source 112 or the current sink 114, respectively. For the second stimulator device 102b, the same current waveform may be received from the current digital-to-analog converter 124. However, the stimulation controller 118 may provide an inverted control (in comparison to control of switches 120, 122 of the first stimulator device 102a) of switches of the second stimulator device 102b, such that the current sink of the second stimulator device 102b may be enabled when the current source 112 of the first stimulator device 102a is enabled, and vice versa. Each of the first stimulator device 102a and the second stimulator device 102b may be connected to a respective electrode 10a, 10b, forming a first pair of electrodes, which may thus receive stimulation current signals that are in anti-phase for forming a stimulation signal that is transmitted into the brain and/or the nerve between the electrodes 10a, 10b.

However, due to circuit mismatch between the current source 112 and the current sink 114 within the first stimulator device 102a, a DC current will be delivered to the tissue via the electrode 10a. This may lead to a safety issue to the electrode 10a and/or the tissue. Such safety issues may arise for all of the stimulator devices 102a, 120b, 102c, 102d.

Charge balancing will now be described for the first stimulator device 102a. Again, it should further be understood that each of the stimulator devices 102a, 102b, 102c, 102d may be implemented in a same manner.

The stimulator device 102a may thus further comprise a compensation unit 140. The compensation unit 140 may be connected between the output node 116 and the output 130 of the stimulation signal generating unit 110. The compensation unit 140 may form a feedback loop for performing charge balancing during output of the stimulation signal, such as during output of a burst in the temporal sequence of bursts of the stimulation signal, by the stimulation signal generating unit 110 to the output 130.

The compensation unit 140 may thus be configured to provide a compensation current signal to the output 130 for compensating an unbalanced charge of the stimulation signal. It should be realized that the compensation unit 140 may not necessarily perfectly balance charge accumulation at the output 130 but may at least reduce charge accumulation so as to avoid risks involved with large offset voltage build-up at the electrode 10a.

The compensation unit 140 may be configured to regulate a common mode voltage of a voltage signal at the output 130 of the stimulator circuit 100. The output 130 may be connected to tissue of a living being via the electrode 10a such that a current signal from the stimulation signal generating unit 110 is converted to the voltage signal.

The stimulator device 102a is particularly adapted for use in applications where a large signal swing at the output 130 is desired. For instance, in peripheral nerve stimulation, the signal swing at the output 130 may preferably be in a range of tens of volts. The stimulation signal generating unit 110 may thus be implemented with a high voltage supply, such as 20 V supply voltage, to provide compliance of the stimulation signal generating unit 110 with large signal swings at the output 130.

The stimulation current provided by the stimulation signal generating unit 110 may be up to a few mA. This may imply that the compensation unit 140 may have a large power consumption.

In order for power consumption of the compensation unit 140 to be limited, the compensation unit 140 may thus comprise an attenuator 142 configured to attenuate the signal at the output 130 of the stimulator circuit 102a such that components of the compensation unit 140 may receive an attenuated stimulation signal.

The compensation unit 140 may further comprise a common mode voltage monitoring element 144, which is configured to monitor the common mode voltage at the output 130. The common mode voltage monitoring element 144 may thus be connected to receive a signal of the output 130 of the stimulator circuit 100, possibly via the attenuator 142.

The common mode voltage monitoring element 144 may be configured to perform extreme point detection of the received signal for determining an unbalance of the common mode voltage. The common mode voltage monitoring element 144 may comprise a maximum point detection element and a minimum point detection element for detecting extreme voltages, upper extreme voltages at local maxima and lower extreme voltages at local minima of the received signal. The common mode voltage monitoring element 144 may further comprise an averaging element for determining an average of the input signal based on the detected local maxima and detected local minima. The averaging element may thus output a representation of the common mode voltage. This is a robust manner of determining the common mode voltage. In addition, the detected extreme voltage of the voltage signal may also be used as information relating to impedance of the electrode-tissue interface as explained further below.

The compensation unit 140 may further comprise a charge balancing element 152 which may be configured to generate the compensation current signal based on the common mode voltage monitored by the common mode voltage monitoring element 144. The compensation unit 140 may be configured to output the compensation current signal to the output 130 of the stimulator circuit 100 for forming a compensated stimulation signal at the output 130. Thus, the stimulation signal in combination with the compensation current signal may form a compensated stimulation signal at the output 130.

The charge balancing element 144 may be configured to generate the compensation current signal based on a difference between the common mode voltage and a reference voltage. Thus, a DC signal may be generated to compensate for any unbalanced charge of the stimulation current signal, such as an unbalanced charge of a burst of the stimulation current signal.

However, due to circuit non-idealities, the compensation unit 140 may not be able to regulate the electrode DC voltage to be exactly equal to the reference voltage during output of the stimulation current signal. This may lead to a remaining offset voltage on the electrode 10a after a burst of the stimulation current signal. Then, when a temporal sequence of bursts is output, the remaining offset voltage will accumulate, and may eventually go above a safety range.

In order to avoid safety issues related to the remaining offset voltage, the stimulator device 102a may be configured to, between bursts of the stimulation current signal, reset the DC voltage at the output 130 to the reference voltage. The stimulator device 102a may thus comprise a switch 132 for selectively connecting the output 130 to the reference voltage. Thus, by closing the switch 132 between bursts of the stimulation signal, any remaining offset voltage may be discharged from the output 130 by resetting the output to the reference voltage.

This may be used in combination with the charge balancing during bursts of the stimulation current signal for ensuring that excessive voltage build-up at the output 130 is avoided.

According to an alternative, the remaining offset voltage may be compensated for by controlling the reference voltage being used by the charge balancing element 152 for determining the compensation current signal. The reference voltage may be adjusted based on the remaining offset voltage after a burst such that the compensation current signal generated by the compensation unit during a next burst will compensate for the remaining offset voltage.

The stimulator device 102a further comprises an impedance monitoring unit 160. The impedance monitoring unit 160 is connected to the output 130. The impedance monitoring unit 160 is configured to, while the stimulation current signal is provided on the output 130, determine information of an impedance relating to the interface between the electrode 10a and tissue.

By using anti-phasic stimulation, crosstalk between the pairs of stimulation devices 102a, 102b, 102c, 102d may be avoided or at least limited. This implies that a well-controlled current path may be provided for each pair of stimulation devices 102a, 102b, 102c, 102d. In other words, a current value that flows through each electrode 10a, 10b, 10c, 10d is well-defined. Since a value of the stimulation current signal is known, it is possible to acquire information that is sufficient for determining the impedance of the electrode-tissue interface.

The impedance monitoring unit 160 is thus configured to determine an extreme voltage at an extreme point of a voltage signal at the output 130 and a phase delay between the voltage signal and the stimulation current signal. By reading out this information, impedance of the electrode-tissue interface may be determined.

The impedance monitoring unit 160 may comprise a voltage detector, which may detect the extreme voltage. However, if the compensation unit 140 is used, the extreme voltage may be determined by the common mode monitoring element 144. The common mode monitoring element 144 may thus communicate the detected extreme voltage to the monitoring unit 160. The monitoring unit 160 may be configured to determine the extreme voltage based on receiving input of the detected extreme voltage. This may include processing the detected extreme voltage by an analog-to-digital converter 162, such that the determined extreme voltage may be output in digital format.

The impedance monitoring unit 160 may receive information of both an upper extreme voltage at an upper extreme point of the voltage signal and a lower extreme voltage at a lower extreme point of the voltage signal from the common mode monitoring element 144. The upper extreme voltage and the lower extreme voltage may be used for providing impedance information of a high resolution. However, it is sufficient to determine one of the extreme voltages and, below, description is made merely relating to the upper extreme voltage being determined.

The impedance monitoring unit 160 may further comprise a counter 164 for determining a time delay representative of the phase delay between the voltage signal and the stimulation current signal.

The counter 164 may be configured to receive a trigger signal from the stimulation controller 118, which generates the waveform to be output by the stimulation signal generating unit 110. For instance, the stimulation controller 118 may provide a trigger to the counter 164 corresponding to an extreme point, such as a peak, of the stimulation current signal. The counter 164 may thus start counting based on receipt of the trigger.

The counter 164 may further receive a clock signal controlling counting by the counter 164 and may increase a counter value by 1 at each pulse of the clock signal. The counter 164 may further receive a trigger to stop counting based on detection of the extreme voltage of the voltage signal. Thus, the counter 164 may be configured to determine the time delay based on determining a time period, number of clock pulses, between the extreme point of the stimulation current signal and the following extreme point of the voltage signal. This may be related to the phase delay via the frequency of the stimulation current signal.

The impedance monitoring unit 160 may be configured to convert the output from the counter to a phase delay or may be configured to output the counter value, which may later be converted to an actual phase delay.

The impedance seen at the output 130 relative to the reference voltage can be represented as a series-connected capacitor and resistor network, having a capacitance $C = C_{ELE1}$, where $C_{ELE1}$ represents double-layer capacitance of the first electrode 10a, and a resistance $R = R_{ELE1} + \alpha \cdot R_{tissue(1,2)}$, where $R_{ELE1}$ represents resistive part of impedance of the first electrode 10a, $\alpha$ is a factor number between 0 and 1 and is related to a relative position of the first electrode 10a and the second electrode 10b, respectively, to the reference electrode 10e, and $R_{tissue(1,2)}$ is tissue resistance between the first electrode 10a and the second electrode 10b.

Similarly, the impedance seen at the output of the second stimulator device 102b relative to the reference voltage can also be represented as a series-connected capacitor and resistor network, having a capacitance $C = C_{ELE2}$, where $C_{ELE2}$ represents double-layer capacitance of the second electrode 10b, and a resistance $R = R_{ELE2} + (1 - \alpha) \cdot R_{tissue(1,2)}$, where $R_{ELE2}$ represents resistive part of impedance of the second electrode 10b.

The factor number $\alpha$ is 0.5 if the reference electrode 10e is arranged exactly in the middle of the first electrode 10a and the second electrode 10b. If the reference electrode 10e is placed closer to the first electrode 10a than the second electrode 10b, $\alpha$ is smaller than 0.5.

During stimulation, the first stimulator device 102a may generate a stimulation current signal having a sinusoidal waveform with an amplitude of $I_a$ and a carrier frequency of $f_{sin_a}$. This implies that a voltage signal $V_1(t)$ with a sinusoidal waveform being phase-shifted in relation to the stimulation current signal will be established on the output 130. The voltage signal $V_1(t)$ may be calculated as:

$$V_1(t) = I_a \cdot e^{j(2\pi f_{sin_a})t} \cdot \left| Z_{1,total} \right| \cdot e^{\varphi_1},$$

where $t$ represents time, $|Z_{1,total}|$ is the modulus of the total impedance of the electrode-tissue interface of the first electrode 10a, and $\varphi_1$ is the phase shift.

Then, the upper extreme voltage of the voltage signal and the phase delay determined by the impedance monitoring unit 160 may be used for determining the impedance.

The impedance monitoring unit 160 may be configured to output the upper extreme voltage of the voltage signal and the phase delay. The stimulator device 102a may further comprise a processor which receives the output from the impedance monitoring unit 160. However, as shown in Fig. 1, the system 100 may comprise a single processor 170, which is configured to receive input from the impedance monitoring units of each of the stimulator devices 102a, 102b, 102c, 102d. Thus, the processor 170 may be configured to determine the impedances relating to the interfaces between each of the electrodes 10a, 10b, 10c, 10d and tissue.

The upper extreme voltage $V_{peak1}$ of the voltage signal at the output 130 of the first stimulator device 102a corresponds to $V_{peak1} = I_a \cdot |Z_{1,total}|$. Further, the phase delay $\varphi_1$ may be calculated based on the time delay $T_{delay1}$ determined by the counter 164 of the impedance monitoring unit 160. The phase delay $\varphi_1$ is given as $\varphi_1 = T_{delay1} \cdot 2\pi f_{sin_a}$.

The processor 170 may thus be configured to calculate the resistive and capacitive parts of the complex impedance of the interface between the first electrode 10a and tissue using the following relations.

The resistive part is given by

$$R_{ELE1} + \alpha \cdot R_{tissue(1,2)} = \frac{V_{peak1}}{I_a \cdot \sqrt{1 + tan^2(\varphi_1)}}.$$

The capacitive part is given by

$$C_{ELE1} = \frac{I_a \cdot \sqrt{1 + tan^2(\varphi_1)}}{V_{peak1} \cdot \tan(\varphi_1) \cdot \left(2\pi \cdot f_{sin_a}\right)}.$$

The impedance of the interfaces for the other three electrodes 10b, 10c, 10d in relation to tissue can be determined in corresponding manner. The stimulation current signal provided by the second stimulator device 102b has a 180° phase shift comparted to the stimulation current signal provided by the first stimulator device 102a.

The stimulation current signals provided by the third stimulator device 102c and the fourth stimulator device 102d will have a different carrier frequency than the stimulation current signals provided by the first stimulator device 102a and the second stimulator device 102b. In addition, the amplitude of the stimulation current signals provided by the third stimulator device 102c and the fourth stimulator device 102d may be different than the amplitude of the stimulation current signals provided by the first stimulator device 102a and the second stimulator device 102b. The respective amplitudes may control a spatial location within the tissue, such as the brain and/or nerve, in which temporal interference stimulation is provided.

The processor 170 may be implemented as a general-purpose processing unit and the system 100 may further comprise software for causing the processor 170 to determine the impedances. The processor 170 may alternatively be implemented as a digital signal processor, which may be suited for the signal processing needed in order to determine the impedances.

However, the processor 170 may alternatively be implemented as a specifically designed processing unit, such as an Application-Specific Integrated Circuit (ASIC) or a Field-Programmable Gate Array (FPGA).

The stimulator device 102a may further comprise a power management unit configured to provide power management of the stimulator device 102a. However, as shown in Fig. 1, the system 100 may comprise a single power management unit 180, which is configured to provide power management for each of the stimulator devices 102a, 102b, 102c, 102d.

The power management unit 180 may be configured to control a supply voltage and the reference voltage to be used by the stimulator devices 102a, 102b, 102c, 102d. The power management unit 180 may be configured to control power usage such that the stimulator devices 102a, 102b, 102c, 102d are power efficient and adapted to an amplitude to be used for the stimulation current signal and the impedance of the electrode-tissue interfaces.

The power management unit 180 may comprise a DC-DC converter which receives a low supply voltage $V_{DDLV}$ used within the system. The DC-DC converter may further be configured to generate a voltage with an increased magnitude based on the low supply voltage $V_{DDLV}$. The DC-DC converter may be configured to output the supply voltage $V_{DDH}$ and the reference voltage $V_{REF}$ to be used by the stimulator devices 102a, 102b, 102c, 102d.

The stimulator devices 102a, 102b, 102c, 102d may need to provide a large signal swing at the output. For instance, in peripheral nerve stimulation, the signal swing at the output may preferably be in a range of tens of volts. The stimulation signal generating unit 110 may thus be implemented with a high voltage supply, such as 20 V supply voltage, to provide compliance of the stimulation signal generating unit 110 with large signal swings at the output 130.

The supply voltage $V_{DDH}$ is thus provided to the stimulation signal generating unit 110 for providing a high voltage supply to support the large swings to be provided at the output 130.

The power management unit 180 may be configured to use a first stimulation current signal (or a first burst of the temporal sequence of bursts of the stimulation current signal) for determining power management settings. The determined power management settings may then ensure an efficient power usage based on the determined power management settings.

Thus, for the first stimulation current signal, the power management unit 180 may be set to output a supply voltage and a reference voltage that should support a worst-case scenario in relation to the amplitude of the stimulation current signal and the impedance of the electrode-tissue interface.

Then, the upper extreme voltage of the voltage signal at the output of each of the stimulator device 102a, 102b, 102c, 102d during the first stimulation current signal is determined and provided as input to the power management unit 180. The power management unit 180 may then determine supply voltage $V_{DDH}$ and the reference voltage $V_{REF}$ to be used based on the upper extreme voltage, such that the power usage of the stimulator devices 102a, 102b, 102c, 102d may be controlled for the next stimulation signal (or the next burst in the temporal sequence of bursts). Thus, the values of the supply voltage $V_{DDH}$ and the reference voltage $V_{REF}$ to be used may be updated by the power management unit 180.

The power management unit 180 may be configured to control a common supply voltage and a common reference voltage for all of the stimulator devices 102a, 102b, 102c, 102d.

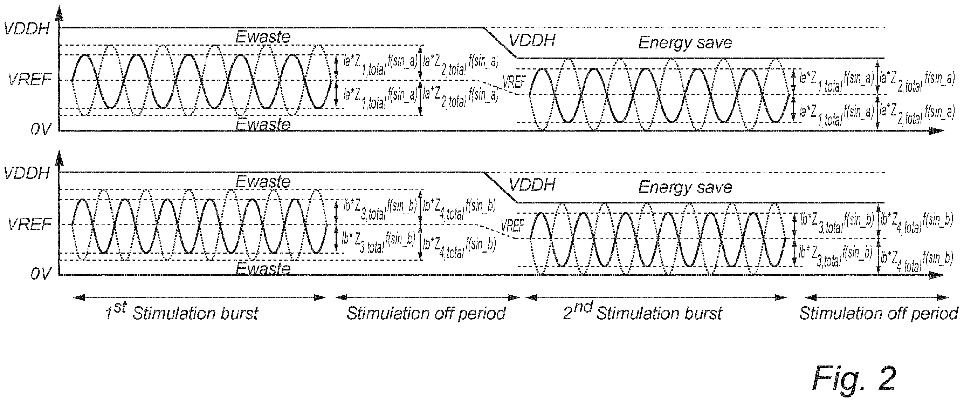

Fig. 2 illustrates, in the upper graph, the voltage signal at the output of the first stimulator device 102a and at the output of the second stimulator device 102b and, in the lower graph, the voltage signal at the output of the third stimulator device 102c and at the output of the fourth stimulator device 102d. As can be seen, there is power wasted during the first stimulation signal (or first burst), as the supply voltage is unnecessarily large.

The power management unit 180 may then be configured to set the supply voltage and the reference voltage for the next stimulation signal (or next burst) in order to save power.

The power management unit 180 may be configured to update the value of the supply voltage $V_{DDH}$ and the reference voltage $V_{REF}$ according to the following rules.

$$V_{REF} = \max\{V_{amp,V1}, V_{amp,V2}, V_{amp,V3}, V_{amp,V4}\} + V_{offset},$$

$$V_{DDH} = 2 * V_{REF},$$

where $V_{amp,V1}$ is an amplitude of the voltage signal at the output of the first stimulator device 102a, $V_{amp,V2}$ is an amplitude of the voltage signal at the output of the second stimulator device 102b, $V_{amp,V3}$ is an amplitude of the voltage signal at the output of the third stimulator device 102c, $V_{amp,V4}$ is an amplitude of the voltage signal at the output of the fourth stimulator device 102d, and $V_{offset}$ is a small offset voltage (e.g., 1V) to provide some margin such that the stimulator devices 102a, 102b, 102c, 102d can still work within compliance.

Then, the stimulator devices 102a, 102b, 102c, 102d start the next stimulation signal (or next burst) with the updated supply voltage $V_{DDH}$ and reference voltage $V_{REF}$ to save power. Peak voltage (and electrode-tissue impedance) information can be extracted optionally in this stimulation signal again, which can serve as input to the power management unit 180 to update the supply voltage $V_{DDH}$ and the reference voltage $V_{REF}$ again for the following stimulation signal (or burst). This procedure may then be repeated, such that even if the electrode-tissue impedance changes over stimulation bursts, the supply voltage $V_{DDH}$ and the reference voltage $V_{REF}$ can still track these changes.

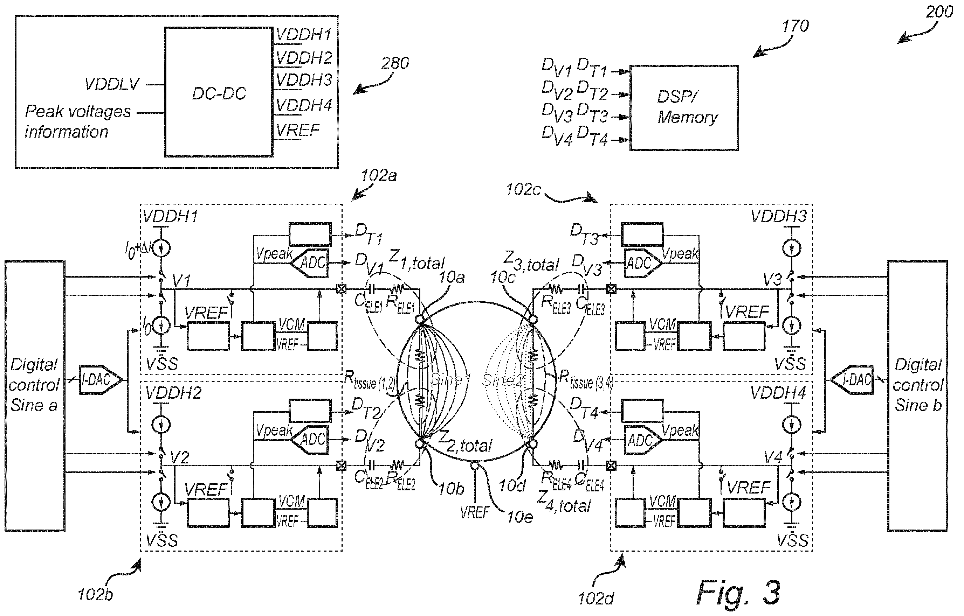

Referring now to Fig. 3, a second embodiment of the power management unit 280 will be described. The supply voltages for the stimulation signal generating units of the stimulator devices 102a, 102b, 102c, 102d may according to the second embodiment be individually controlled. Thus, the stimulator devices 102a, 102b, 102c, 102d may be configured to receive different supply voltages to the stimulation signal generating units. Apart from this, the system 200 is identical to the system 100 described above and the identical features are not further discussed here.

Considering that the amplitudes of the voltage signals at the outputs of the different stimulator devices 102a, 102b, 102c, 102d can be different (due to different impedance and stimulation current amplitudes), an even more energy efficient option may be provided as discussed below.

Instead of providing one common supply voltage for all the four stimulator devices 102a, 102b, 102c, 102d, the power management unit 280 can provide four different supply voltages $V_{DDH1}$, $V_{DDH2}$, $V_{DDH3}$, $V_{DDH4}$ for the four stimulator devices 102a, 102b, 102c, 102d, such that each four stimulator device 102a, 102b, 102c, 102d receives its own supply voltage $V_{DDH1}$, $V_{DDH2}$, $V_{DDH3}$, $V_{DDH4}$, respectively, which is customized by its respective upper extreme voltage.

However, the stimulator device 102a, 102b, 102c, 102d may still share one common reference voltage $V_{REF}$ together with the tissue bias voltage provided to the reference electrode 10e for safety considerations.

The power management unit 280 may be configured to update the value of the supply voltage $V_{DDH}$ and the reference voltage $V_{REF}$ according to the following rules.

$$V_{REF} = \max\{V_{amp,V1}, V_{amp,V2}, V_{amp,V3}, V_{amp,V4}\} + V_{offset},$$

$$V_{DDH1} = V_{REF} + V_{amp,V1} + V_{offset},$$

$$V_{DDH2} = V_{REF} + V_{amp,V2} + V_{offset},$$

$$V_{DDH3} = V_{REF} + V_{amp,V3} + V_{offset},$$

$$V_{DDH4} = V_{REF} + V_{amp,V4} + V_{offset}.$$

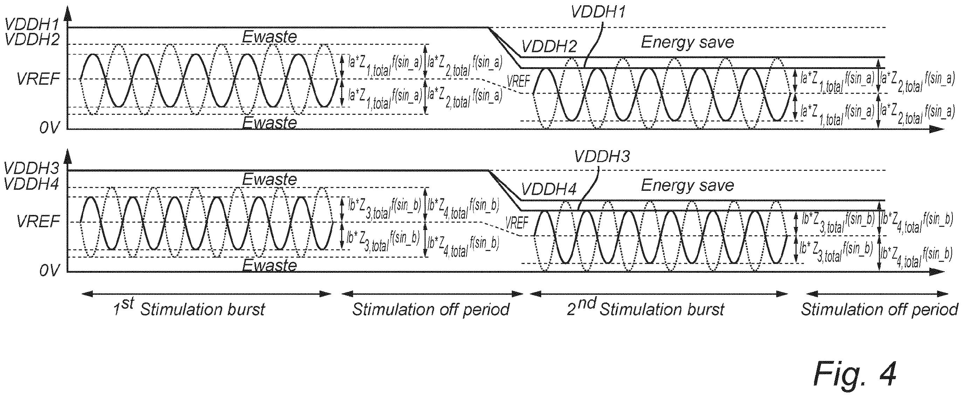

Fig. 4 illustrates in the upper graph, the voltage signal at the output of the first stimulator device 102a and at the output of the second stimulator device 102b and, in the lower graph, the voltage signal at the output of the third stimulator device 102c and at the output of the fourth stimulator device 102d. The voltage signals for a first stimulation signal (or first burst) and the voltage signals for a second stimulation signal (or second burst) are illustrated, wherein the supply voltages $V_{DDH1}$, $V_{DDH2}$, $V_{DDH3}$, $V_{DDH4}$ are individually controlled. As can be seen, the power management unit 280 may provide even more efficient power saving compared to the power management unit 180 of the first embodiment.

Above, two pairs of electrodes and associated stimulator devices are described. It should be realized that the systems described above may be extended to use of further pairs of electrodes with associated stimulator devices.

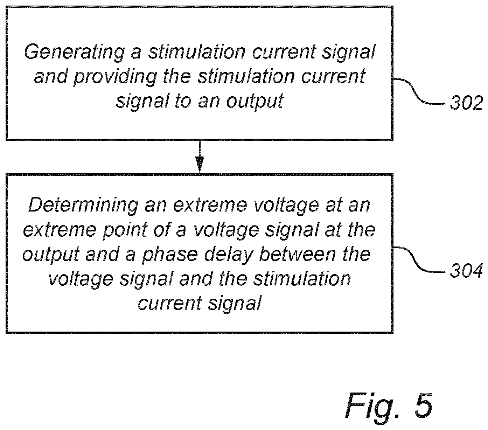

Referring now to Fig. 5, a method for monitoring a stimulation signal will be briefly described.

The method comprises: generating 302 a stimulation current signal and providing the stimulation current signal to an output of the stimulation signal generating unit, wherein the stimulation current signal comprises a sinusoidal-like waveform.

The method may further comprise detecting an upper extreme voltage of a voltage signal at the output and detecting a lower extreme voltage of the voltage signal. The detected upper and lower extreme voltages may be used for determining a common mode voltage.

The method may further comprise generating a compensation current signal for charge balancing based on comparing the common mode voltage to a reference voltage, wherein the compensation current signal may be provided to the output for compensating an unbalanced charge of the stimulation current signal and forming a compensated stimulation signal at the output. The method may thus ensure that build-up of an offset voltage at the output is avoided during output of the stimulation current signal.

The method further comprises, while the stimulation current signal is provided on the output, determining 304 an extreme voltage at an extreme point of a voltage signal at the output and a phase delay between the voltage signal and the stimulation current signal for determining information of an impedance relating to an interface between an electrode, connected to the output, and tissue.

The determining of the extreme voltage may be based on receiving input of the detected upper and/or lower extreme voltages used in charge balancing.

The method may further comprise outputting the information of the extreme voltage and the phase delay to a processor and determining, by the processor, the impedance of the interface between the electrode and tissue using knowledge of an amplitude and frequency of the stimulation current signal.

The method may further comprise providing power management for generation of the stimulation current signal, wherein a supply voltage is controlled based on the determined extreme voltage, wherein the supply voltage controls generation of the stimulation current signal by the stimulation signal generating unit.

In the above the inventive concept has mainly been described with reference to a limited number of examples. However, as is readily appreciated by a person skilled in the art, other examples than the ones disclosed above are equally possible within the scope of the inventive concept, as defined by the appended claims.

**Claims**

1. A stimulator device (102a; 102b; 102c; 102d) for providing a stimulation signal, said stimulator device (102a; 102b; 102c; 102d) comprising:

   a stimulation signal generating unit (110) configured to generate a stimulation current signal and provide the stimulation current signal to an output (130) of the stimulation signal generating unit (110), wherein the stimulation current signal comprises a sinusoidal-like waveform; and
   an impedance monitoring unit (160) connected to the output (130) of the stimulation signal generating unit (110), wherein the impedance monitoring unit (160) is configured to, while the stimulation current signal is provided on the output (130) of the stimulation signal generating unit (110), determine an extreme voltage at an extreme point of a voltage signal at the output (130) and a phase delay between the voltage signal and the stimulation current signal for determining information of an impedance relating to an interface between an electrode (10a; 10b; 10c; 10d), connected to the output (130), and tissue.

2. The stimulator device according to claim 1, further comprising an electrode (10a; 10b; 10c; 10d) connected to the output (130) of the signal generating unit (110) for receiving the stimulation current signal, said electrode (10a; 10b; 10c; 10d) being configured for connection to tissue for providing the stimulation current signal into a body part.

3. The stimulator device according to claim 1 or 2, further comprising a processor (170) configured to receive the extreme voltage and the phase delay and configured to determine the impedance based on the received extreme voltage and phase delay and knowledge of an amplitude and frequency of the stimulation current signal.

4. The stimulator device according to any one of the preceding claims, further comprising a compensation unit (140) configured to generate a compensation current signal for charge balancing and provide the compensation current signal to the output (130) of the stimulation signal generating unit (110) for compensating an unbalanced charge of the stimulation current signal and forming a compensated stimulation signal at the output (130) of the stimulation signal generating unit (110).

5. The stimulator device according to claim 4, wherein the compensation unit (140) comprises an extreme voltage detector (144) for detecting the extreme voltage of the voltage signal, wherein the impedance monitoring unit (160) is configured to receive the detected extreme voltage from the compensation unit (140) for determining the extreme voltage.

6. The stimulator device according to any one of the preceding claims, wherein the impedance monitoring unit (160) comprises a counter (164) for determining a time delay representative of the phase delay.

7. The stimulator device according to any one of the preceding claims, wherein the impedance monitoring unit (160) being configured to determine the extreme voltage comprises the impedance monitoring unit (160) being configured to determine both an upper extreme voltage at an upper extreme point of the voltage signal and a lower extreme voltage at a lower extreme point of the voltage signal.

8. The stimulator device according to any one of the preceding claims, wherein the stimulation current signal comprises a sequence of temporally separated bursts, wherein each burst comprises an alternating current, AC, signal having the sinusoidal-like waveform.

9. The stimulator device according to claim 8, wherein the stimulator device (102a; 102b; 102c; 102d) is configured to, between bursts of the stimulation current signal, reset a direct current, DC, voltage at the output (130) of the stimulation signal generating unit (110) to a reference voltage.

10. The stimulator device according to claim 8 or 9, further comprising a power management unit (180; 280) configured to control a supply voltage controlling generation of the stimulation current signal by the stimulation signal generating unit (110) based on the determined extreme voltage.

11. A system (100; 200) for providing temporal interference stimulation of a brain and/or a nerve of a living being, said system (100; 200) comprising:

   a first pair of electrodes (10a, 10b) configured to be arranged in a first relation to the brain and/or the nerve;
   a second pair of electrodes (10c, 10d) configured to be arranged in a second relation to the brain and/or the nerve;
   wherein each electrode of the first pair of electrodes (10a, 10b) and the second pair of electrodes (10c, 10d) forms part of a respective stimulator device (102a; 102b; 102c; 102d) according to any one of the preceding claims.

12. The system according to claim 11, further comprising a reference electrode (10e) for defining a reference voltage in relation to each electrode of the first pair of electrodes (10a, 10b) and the second pair of electrodes (10c, 10d).

13. The system according to claim 11 or 12, wherein the system (100) comprises a common power management unit (180) for controlling a common supply voltage of the stimulation signal generating units of each of the stimulator devices (102a; 102b; 102c; 102d).

14. The system according to claim 11 or 12, wherein a respective supply voltage controlling generation of the stimulation current signal by the respective stimulation signal generating units (102a; 102b; 102c; 102d) is individually controlled.

15. A method for monitoring a stimulation signal, said method comprising:

   generating (302) a stimulation current signal and providing the stimulation current signal to an output of a stimulation signal generating unit, wherein the stimulation current signal comprises a sinusoidal-like waveform;
   while the stimulation current signal is provided on the output of the stimulation signal generating unit, determining (304) an extreme voltage at an extreme point of a voltage signal at the output and a phase delay between the voltage signal and the stimulation current signal for determining information of an impedance relating to an interface between an electrode, connected to the output, and tissue.

*Fig. 1*

*Fig. 2*

Fig. 3

EP 4 548 962 A1

Fig. 4

EP 4 548 962 A1

Generating a stimulation current signal and providing the stimulation current signal to an output

— 302

Determining an extreme voltage at an extreme point of a voltage signal at the output and a phase delay between the voltage signal and the stimulation current signal

— 304

*Fig. 5*

Europäisches
Patentamt
European
Patent Office
Office européen
des brevets

# EUROPEAN SEARCH REPORT

**Application Number**

EP 23 20 7588

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/023946 A1 (VALVANO JONATHAN W [US] ET AL) 24 January 2013 (2013-01-24) | 1-3,6,7, 15 | INV. A61N1/36 |
| Y | * paragraphs [0038] - [0039], [0078]; figures 4,5 * | 4,5,8-14 | |
| Y | US 2016/310741 A1 (BARU MARCELO [US] ET AL) 27 October 2016 (2016-10-27) * paragraphs [0002], [0065], [0079]; claims 1,7 * | 4,5,8-14 | |
| Y | US 2016/045743 A1 (LIU WENTAI [US] ET AL) 18 February 2016 (2016-02-18) * paragraph [0046] * | 4,5 | |
| A | AU 2013 207 558 A1 (SECOND SIGHT MEDICAL PROD INC) 1 August 2013 (2013-08-01) * paragraphs [0064] - [0066], [0076] * | 1-15 | |
| A | US 2021/138232 A1 (PAZ SHACHAR [IL] ET AL) 13 May 2021 (2021-05-13) * the whole document * | 1-15 | |

| | |
|---|---|
| | **TECHNICAL FIELDS SEARCHED (IPC)** |
| | A61N |

The present search report has been drawn up for all claims

| Place of search | Date of completion of the search | Examiner |
|---|---|---|
| Munich | 21 March 2024 | Aronsson, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...................................................................

& : member of the same patent family, corresponding document

EPO FORM 1503 03.82 (P04C01)

**EP 4 548 962 A1**

## ANNEX TO THE EUROPEAN SEARCH REPORT
## ON EUROPEAN PATENT APPLICATION NO.

EP 23 20 7588

This annex lists the patent family members relating to the patent documents cited in the above-mentioned European search report.
The members are as contained in the European Patent Office EDP file on
The European Patent Office is in no way liable for these particulars which are merely given for the purpose of information.

21-03-2024

| Patent document cited in search report | | | Publication date | Patent family member(s) | | | Publication date |
|---|---|---|---|---|---|---|---|
| US 2013023946 | A1 | | 24-01-2013 | EP | 2691019 | A1 | 05-02-2014 |
| | | | | JP | 2014514054 | A | 19-06-2014 |
| | | | | JP | 2017080505 | A | 18-05-2017 |
| | | | | US | 2013023946 | A1 | 24-01-2013 |
| | | | | US | 2016262653 | A1 | 15-09-2016 |
| | | | | US | 2019357805 | A1 | 28-11-2019 |
| | | | | WO | 2012134891 | A1 | 04-10-2012 |
| US 2016310741 | A1 | | 27-10-2016 | EP | 3085414 | A1 | 26-10-2016 |
| | | | | US | 2016310741 | A1 | 27-10-2016 |
| US 2016045743 | A1 | | 18-02-2016 | EP | 2961477 | A1 | 06-01-2016 |
| | | | | US | 2016045743 | A1 | 18-02-2016 |
| | | | | WO | 2014134075 | A1 | 04-09-2014 |
| AU 2013207558 | A1 | | 01-08-2013 | AU | 2011211414 | A1 | 01-09-2011 |
| | | | | AU | 2013207558 | A1 | 01-08-2013 |
| US 2021138232 | A1 | | 13-05-2021 | CN | 112867533 | A | 28-05-2021 |
| | | | | EP | 3801746 | A2 | 14-04-2021 |
| | | | | ES | 2948813 | T3 | 19-09-2023 |
| | | | | IL | 280015 | A | 01-03-2021 |
| | | | | US | 2021138232 | A1 | 13-05-2021 |
| | | | | US | 2023001181 | A1 | 05-01-2023 |
| | | | | WO | 2020035852 | A2 | 20-02-2020 |

EPO FORM P0459

For more details about this annex : see Official Journal of the European Patent Office, No. 12/82